(19) Europäisches Patentamt
European Patent Office
Office européen des brevets

(11) Veröffentlichungsnummer: **0 416 279 A1**

(12) **EUROPÄISCHE PATENTANMELDUNG**

(21) Anmeldenummer: 90114591.2

(22) Anmeldetag: 30.07.90

(51) Int. Cl.5: **C07C 37/00**, C07C 39/07

(30) Priorität: 09.08.89 DE 3926290

(43) Veröffentlichungstag der Anmeldung:
13.03.91 Patentblatt 91/11

(84) Benannte Vertragsstaaten:
BE DE ES FR GB

(71) Anmelder: BASF Aktiengesellschaft
Carl-Bosch-Strasse 38
W-6700 Ludwigshafen(DE)

(72) Erfinder: Steck, Werner, Dr.
Auerstrasse 4
W-6700 Ludwigshafen(DE)
Erfinder: Lermer, Helmut, Dr.
Bockenheimer Strasse 12
W-6700 Ludwigshafen(DE)
Erfinder: Schwarzmann, Mathias, Dr.
Carl-Bosch-Strasse 54
W-6703 Limburgerhof(DE)
Erfinder: Dokner, Toni, Dr.
Grossgasse 6
W-6701 Meckenheim(DE)

(54) **Verfahren zur Herstellung von Kresolisomergemischen mit einem Molverhältnis von para- zu meta-Kresol von mindestens 0,6:1 bis 10:1.**

(57) Verfahren zur Herstellung von Kresolisomerengemischen mit einem Molverhältnis von para- zu meta-Kresol von 0,6:1 bis 10:1 durch Isomerisierung von Kresolgemischen oder ortho- und/oder meta-Kresol an Zeolithkatalysatoren des Pentasil-Typs in der Gasphase bei Temperaturen von 300 bis 600 °C und Drücken von 0,01 bis 50 bar, indem die mittlere Teilchengröße des Zeolithkatalysators mindestens 3 μm beträgt und die im festgelegten Test bei der Disproportionierung von Ethylbenzol zu Diethylbenzolisomeren eine Selektivität für para-Diethylbenzol von mindestens 60 % erreichen.

# VERFAHREN ZUR HERSTELLUNG VON KRESOLISOMERENGEMISCHEN MIT EINEM MOLVERHÄLTNIS VON PARA- ZU META-KRESOL VON MINDESTENS 0,6:1 BIS 10:1

Die vorliegende Erfindung betrifft ein Verfahren zur Isomerisierung von Kresolen, insbesondere o-Kresol, an Zeolithkatalysatoren zu Isomerengemischen, in denen das Molverhältnis von p-Kresol zu m-Kresol mindestens 0,6:1 beträgt.

Aus GB-A-2 012 271 ist ein Verfahren zur katalytischen Isomerisierung von o-Kresol an Aluminiumsilikatzeolithen bekannt, welches bestenfalls ein p-/m-Kresol-Molverhältnis von 0,41:1 erbringt.

Aus der US-A-4 503 269 ist ein Verfahren zur Isomerisierung von o-Kresol zu einem Gemisch aus meta- und para- sowie die Isomerisierung von m-Kresol zu p-Kresol mit wenig o-Kresol an Aluminosilikatzeolithen in Gegenwart von 1 bis 10 Mol Wasserstoff je Mol Kresol bekannt. Das Verfahren liefert trotz der Anwesenheit von Wasserstoff bestenfalls ein p-/m-Kresol-Molverhältnis von nur 0,35:1 im Produktaustrag.

Aus der US-A-4 691 063 ist ferner ein Verfahren zur Isomerisierung von Kresolen an phosphorhaltigen Aluminosilikat-Zeolithen mit 1 bis 8 Gew.-% Phosphor bei 350 bis 500°C und 1 bis 60 atm bekannt. Dieses Verfahren an Phosphor-modifizierten Zeolithen liefert bestenfalls einen Isomerenaustrag von p-/m-Kresol im Molverhältnis von 0,46:1.

Folglich sind bislang durch Isomerisierung von Kresolen nur ternäre Isomerengemische erhältlich, die ein p-/m-Kresol-Molverhältnis von bestenfalls 0,46:1 aufweisen. Nach der destillativen Abtrennung von o-Kresol verbleiben daher binäre Gemische von p- und m-Kresol mit einem Molverhältnis von para-/meta- von gleichfalls nur maximal 0,46:1.

Angesichts der Siedepunktslagen von 191,1°C für o-Kresol, 202,5°C für p-Kresol und 202,7°C für m-Kresol ist es leicht verständlich, daß eine destillative Trennung von p-Kresol und m-Kresol nur schwer möglich ist.

Um den steigenden Bedarf an p-Kresol zu decken, bestand daher die Aufgabe durch Isomerisierung von reichlich vorhandenen Kresolisomeren, beispielsweise dem leicht zugänglichen o-Kresol oder z.B. technischen Kresol, Isomerengemische mit einem hohen Gehalt an p-Kresol herzustellen und somit die anschließend Isomerentrennung wirtschaftlicher zu gestalten.

Demgemäß wurde ein Verfahren zur Herstellung von Kresolisomerengemischen mit einem Molverhältnis von para- zu meta-Kresol von 0,6:1 bis 10:1 durch Isomerisierung von Kresolgemischen oder ortho- und/oder meta-Kresol an Zeolithkatalysatoren des Pentasil-Typs in der Gasphase bei Temperaturen von 300 bis 600°C und Drücken von 0,01 bis 50 bar, gefunden, welches dadurch gekennzeichnet ist, daß die mittlere Teilchengröße des Zeolithkatalysators mindestens 3 $\mu$m beträgt und die im festgelegten Test bei der Disproportionierung von Ethylbenzol zu Diethylbenzolisomeren eine Selektivität für para-Diethylbenzol von mindestens 60 % erreichen.

Die für die erfindungsgemäße Isomerisierung geeigneten Reaktionsbedingungen liegen bei 300 bis 600°C, vorzugsweise bei 350 bis 520°C und Drücken von 0,01 bis 50 bar, vorzugsweise 0,1 bis 5 bar, besonders bevorzugt bei Atmosphärendruck von ca. 1 bar. Die Reaktion läßt sich beispielsweise in der Gasphase bei Normaldruck in einem Festbett, Fließ-oder Wirbelbett ausführen. Bei einer Gesamtgewichtsbelastung WHSV von 1 bis 15 h$^{-1}$ (Dimension der WHSV = g Einsatzgemisch an Kresolen je g Katalysator und Stunde) lassen sich gute Ergebnisse erzielen. Es hat sich dabei als vorteilhaft erwiesen die Reaktion in Gegenwart von Inertgasen wie Stickstoff oder Edelgasen durchzuführen.

Es wurde gefunden, daß die Kristallgröße des Zeolithkatalysators einen großen Einfluß auf das erreichbare p-Kresol : m-Kresol-Verhältnis ausübt und para-Kresol-reiche Isomerengemische insbesondere mit Kristallen erhältlich sind, deren Größe mindestens etwa 3 $\mu$m wie 3 bis 500 $\mu$m, vorzugsweise 20 bis 50 $\mu$m besonders bevorzugt 3 bis 25 $\mu$m bzw. 3 bis 15 $\mu$m beträgt. Die Kristallgröße läßt sich gemäß dem Stand der Technik leicht elektronenmikroskopisch entweder in Transmission (TEM) oder mit REM (Rasterelektronenmikroskopie) bestimmen.

Zur Charakterisierung der erfindungsgemäßen Zeolith-Katalysatoren durch eine Testreaktion eignet sich die in der Literatur beschriebene Ethylbenzol-Disproportionierung (EBD-Test s. H.G. Karge, Z. Sarbak, K. Hatada, J. Weitkamp, P.A. Jacobs, J. Catal. 82 (1983) 236).

Die Testreaktion wird in einem Rohrreaktor mit einem Innendurchmesser von 6 mm durchgeführt. das 90 cm lange Edelstahlrohr ist zu einer Wendel gebogen. Am Reaktorende verhindert ein Sieb den Austrag von Katalysator. Die Beheizung des Reaktors erfolgt durch einen Warmluftofen. Ein Stickstoffstrom von 10 Nl h$^{-1}$ wird in einem Sättiger mit Ethylbenzol beladen. Der Sättiger ist ein auf 25°C thermostatisiertes Doppelmantelglasrohr. Das innere Glasrohr des Sättigers ist mit einem hochporösen, inerten Material gefüllt, welches mit Ethylbenzol benetzt wird. Der von unten nach oben strömende Stickstoff sättigt sich entlang der Schüttung entsprechend dem eingestellten Partialdruck (13 hPa bei 25°C) mit dem Ethylbenzol auf. Das resultierende Gemisch wird danach in absteigender Fahrweise bei 250°C über das Katalysatorbett geleitet. Der Katalysator wird dazu als Splitt von 1 bis 2 mm Durchmesser in einer Menge von 1 g eingesetzt. Die Reaktorausträge werden kondensiert und zur gaschromatographischen Analyse gesammelt. Selbstverständlich kann die Analyse automatisiert und on-line erfolgen.

Zunächst stellt der Umsatz des Ethylbenzols ein direktes Maß für die Brönsted-Acidität des Zeolithen dar. Das gebildete Diethylbenzol kann in drei Isomeren, nämlich o-, m- oder p-Diethylbenzol vorliegen. Die Bildung des p-Diethylbenzols wiederum dient der Quantifizierung der para-Selektivität der untersuchten Zeolith-Probe. Erreicht im EBD-Test bei gegebenen Bedingungen die gebildete Menge an p-Diethylbenzol mindestens einen Anteil von 60 % im Isomerengemisch der Diethylbenzole, so ist der Katalysator auch bestens für die erfindungsgemäße Isomerisierung der Kresole zu einem an para-Kresol reichen Isomerengemisch geeignet und damit ein Merkmal der erfindungsgemäßen Katalysatoren. Im Reaktionsaustrag der Testreaktion liegen häufig nur Ethylbenzol, Benzol und die Diethylbenzole vor, deren gaschromatographische Analytik mit geringem Aufwand durchführbar ist.

Die für das erfindungsgemäße Verfahren geeigneten Zeolithe vom Pentasiltyp weisen als sekundäre Struktureinheit (= Secondary Building Unit, Grundbaustein der Zeolith-Struktur) einen aus $TO_4$-Tetraedern aufgebauten Fünfring auf, wobei als T-Kation Si, Al oder Ga verwendet werden können. Die Definition der Pentasil-Zeolithe stammt von G.T. Kokotailo und W.M. Meier, Chem. Soc. Spec. Publ. 1980, 33, Seite 133 - 139. Pentasilzeolithe sind durch ein hohes molares $SiO_2/Al_2O_3$-Verhältnis - auch Modul genannt - gekennzeichnet, sowie durch Porengrößen, die zwischen denen der Zeolithe vom Typ A und denen vom Typ X oder Y liegen. Für das erfindungsgemäße Verfahren haben sich beispielsweise Pentasil-Zeolithe mit $SiO_2/Al_2O_3$-Verhältnissen zwischen 25 und 700 als gut geeignet erwiesen. Die Herstellung dieser Pentasil-Zeolithe wird u.a. in den US-A-3 702 886 (ZSM-5), US-A-3 709 979 (ZSM-11) und US-A-4 061 724 (Silicalite®) beschrieben. Auch gehören hierzu die isotaktischen Pentasil-Zeolithe nach EP-A-0 034 727.

Der Aluminosilikatzeolith wird z.B. aus einer Aluminiumverbindung, vorzugsweise $Al(OH)_3$ oder $Al_2(SO_4)_3$ und einer Siliciumkomponente, wie hochdisperses Siliciumdioxid, Kieselgel oder Kieselsol in wäßriger Aminlösung, insbesondere in Polyaminen wie 1,6-Diaminohexan oder 1,3-Diaminopropan oder Triethylentetramin-Lösung mit oder ohne Alkali- oder Erdalkalizusatz bei 100 bis 220°C unter autogenem Druck hergestellt. Die erhaltenen Aluminosilikatzeolithe enthalten je nach Wahl der Einsatzstoffmengen ein $SiO_2/Al_2O_3$-Verhältnis von 25 bis 700.

Auch Galliumsilikatzeolithe oder Zeolithe, welche als dreiwertiges Gitterion gleichzeitig Gallium und Aluminium enthalten, sind geeignet.

Geeignete Katalysatoren erhält man auch dadurch, daß man in einem ersten Syntheseschritt gemäß dem Stand der Technik zuerst Zeolithe mit großen Kristallen herstellt, die zunächst katalytisch nicht ausreichend aktiv sind und diese dann durch Substitution des Siliciums durch Aluminium oder Gallium nachträglich für die Isomerisierung ausreichend aktiviert. So können beispielsweise weitgehendst Aluminium-freie Zeolithe nach US-A-3 702 886 oder Borzeolithe nach EP-A-7 081 oder US-A-4 268 420 in Form ausreichend großer Kristalle hergestellt und anschließend gemäß dem Stand der Technik aluminiert werden. Die Aluminierung kann sowohl in der Gasphase bei erhöhter Temperatur mit $AlCl_3$ (M.W. Anderson et al, J. Chem. Soc., Chem. Commun., 1984, p. 157) oder in flüssiger Phase sowohl bei Normaldruck bis 100°C oder auch unter autogenem Druck bei Temperaturen bis 180°C vorgenommen werden. Als Al-Verbindungen können Salze wie Chloride oder Nitrate des Aluminiums in Form ihrer wässrigen Lösungen eingesetzt werden. Dabei kann der pH-Wert im Bereich zwischen 2,5 und 13 liegen. Wird Aluminium im alkalischen Milieu in das Gitter insertiert, so können als Al-Quellen auch wäßrige Lösungen von $KAlO_2$ oder $NaAlO_2$ verwendet werden (X. Liu et al, J. Chem. Soc., Chem. Commun., 1986, p. 582). Die Pentasilzeolithe können zu diesen Aktivierungen als Pulver oder nach Abbrennen eines geeigneten Bindemittels auch als Stränge oder Tabletten eingesetzt werden.

Geeignete Pentasilzeolithe können auch dadurch erhalten werden, daß in zu stark aciden Katalysatoren ein Teil des Aluminiums aus dem Gitter entfernt oder durch Silicium ersetzt wird. Dies kann durch die bekannten hydrothermalen Methoden in Gegenwart von Wasserdampf mit meist anschließender Behandlung mit einer Säure, durch Extraktion mit Komplexbildnern wie EDTA oder durch Behandlung mit $SiCl_4$ bei erhöhter Temperatur in der Gasphase erfolgen. Eine neuere Methode ist die Behandlung der Zeolithe mit einer wäßrigen Lösung von $(NH_4)_2SiF_6$ (D.W. Breck, G.W. Skeels, A.C.S., Symp. Ser., v. 218, 1983; EP-A-0 082 211). Desgleichen kann die Oberflächenacidität der Zeolithe durch Calcination in Gegenwart von Alkaliionen beseitigt oder stark abgesenkt werden.

Die pulverförmig anfallenden Zeolithe können nach ihrer Synthese, Isolierung, Trocknung bei 100 bis 160°C und Calcinierung bei 400 bis 550°C, mit einem Bindemittel im Massenverhältnis Zeolith zu Bindemittel 90:10 bis 40:60 Gew.-% zu Formkörpern wie Strängen oder Tabletten verformt werden. Als Bindemittel eignen sich u.a. diverse Siliciumdioxide wie hochdisperses $SiO_2$, diverse Aluminiumoxide wie

3

Boehmit, Gemische aus $SiO_2$ und $Al_2O_3$ oder Ton, amorphe Aluminosilikate oder $TiO_2$. Nach der Verformung werden die Extrudate oder Preßlinge bei 110°C getrocknet und bei Temperaturen um 500°C calciniert.

Man erhält auch gut geeignete Katalysatoren, wenn der isolierte Zeolith direkt nach der Trocknung verformt wird und erst nach der Verformung einer Calcinierung unterworfen wird. Die Pentasilzeolithe können in reiner Form, ohne Binder, als Stränge oder Tabletten eingesetzt werden, wobei als Verstrangungs- oder Peptisierungsmittel z.B. Ethylcellulose, Stearinsäure, Kartoffelstärke, Ameisensäure, Oxalsäure, Essigsäure, Salpetersäure, Ammoniak, Amine und Graphit oder deren Gemische verwendet werden können.

Liegt der Zeolith aufgrund der Art seiner Herstellung vor seiner Applikation nicht in der katalytisch aktiven, aciden H-Form vor, sondern z.B. in der Na-Form, dann kann diese durch Ionenaustausch, z.B. mit Ammoniumionen, und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 550°C zu regenerieren. Die Zeolithe erhalten dadurch in der Regel ihre Anfangsaktivität zurück.

Um eine möglichst hohe Selektivität, hohen Umsatz sowie lange Standzeiten zu erreichen, ist es gegebenenfalls vorteilhaft, die Zeolithe zu modifizieren, wobei der Gehalt an den Modifizierungselementen so zu bemessen ist, daß eine ausreichende Aktivität der Katalysatoren gewährleistet ist.

Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle werden beispielsweise Edelmetalle, wie Pd oder Pt, eingesetzt.

Zweckmäßigerweise führt man die Dotierung so durch, daß man z.B. den verformten Zeolithen in einem Steigrohr vorlegt und bei 20 bis 100°C, z.B. eine wäßrige oder ammoniakalische Lösung eines Halogenids oder eines Nitrats der voranbeschriebenen Metalle überleitet. Ein derartiger Ionenaustausch kann z.B. an der Wasserstoff-, Ammonium-, Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat, einem Carbonat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Die nachstehend beschriebenen Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelgut eingesetzt werden.

Kresole sind wichtige Zwischenprodukte zur Herstellung von Riechstoffen und Pflanzenschutzmitteln. Besonderes Interesse besteht für para-Kresol, das beispielsweise zur Herstellung von Antioxidantien benötigt wird (vgl. jeweils: Ullmanns Encyklopädie der technischen Chemie, 4. Aufl., Bd. 15, S. 72, Verlag Chemie, Weinheim, 1978).

Beispiele

Isomerisierung von Kresolen

Beispiele 1 bis 22 und Vergleichsbeispiele V 1 bis V 4.

Gasphasenreaktionen zur Isomerisierung von o-Kresol

Die Reaktionen in der Gasphase wurden bei Normaldruck unter isothermen Bedingungen in einem Rohrreaktor (Wendel, 0,6 cm Innendurchmesser, Rohrlänge 90 cm) im einmaligen Durchgang an einem Katalysatorfestbett bei 250 bis 500°C durchgeführt. Die Katalysatoreinwaage betrug zwischen 2 und 10 g. Die Reaktionsprodukte wurden durch Gaschromatographie analysiert.

Zur Herstellung von Kresol-Isomerengemischen im vorstehend beschriebenen Rohrreaktor wurden die Einsatzprodukte, z.B. o-Kresol, bei Normaldruck mit einer Dosierpumpe dem Reaktor kontinuierlich zugeführt und dann gasförmig mit einem Trägergasstrom von beispielsweise 5 bis 30 Normliter/h Stickstoff über den Katalysator geleitet. Das mit Katalysator beschickte Festbett wird dabei je nach Fahrweise entweder aufsteigend oder absteigend von den gasförmig vorliegenden Edukten und Reaktionsprodukten durchströmt.

Nach beendeter Isomerisierung wird der Reaktionsaustrag kondensiert und nach der in Stunden [h] angegebenen Laufzeit gaschromatisch analysiert. Die Umsätze (U) und Selektivitäten (S) sind in den Tabellen in Flächenprozenten angegeben.

Tabelle 1: Isomerisierung von o-Kresol

Isomerisierung von o-Kresol mit 4 g Katalysator; $N_2$ (l/h) = Stickstoffstrom in Normliter/h; Ed = Einsatzprodukt o-Kresol in ml/h. U-oK (6 h) = Umsatz an o-Kresol nach 6 Stunden, p/m (6 h) = Verhältnis von p-Kresol zu m-Kresol im Produktaustrag nach 6 Stunden; S (K) = Selektivität p-Kresol + m-Kresol als Maß für die Selektivität für die gewinnbaren Kresolisomeren bzw. Maß des Verlustes an o-Kresol zu Nebenprodukten.

| Beispiel | T °C | $N_2$ 1/h | Ed | U-ok (6 h) | p/m (6 h) | S(K) p+m% | Katalysator Nr. |
|---|---|---|---|---|---|---|---|
| 1 | 450 | 10 | 10 | 37,9 | 0,60 | 61,2 | 1 |
| 2 | 250 | 10 | 10 | 1,3 | 0,88 | 57,0 | 2 |
| 3 | 350 | – | – | 10,9 | 0,99 | 78,8 | 2 |
| 4 | 450 | – | – | 11,8 | 0,88 | 79,4 | 2 |
| 5 | 450 | – | 20 | 11,9 | 0,81 | 92,8 | 2 |
| 6 | 450 | – | 10 | 15,5 | 0,71 | 85,7 | 2 |
| 7 | – | 10 | – | 7,5 | 1,20 | 90,5 | 2 |
| 8 | – | – | – | 14,6 | 0,67 | 95,5 | 2A |
| 9 | – | – | – | 11,8 | 0,72 | 95,6 | 2B |
| 10 | – | – | – | 8,5 | 1,15 | 89,2 | 2C |
| 11 | – | – | – | 8,8 | 1,06 | 87,9 | 2D |
| 12 | 450 | 10 | 10 | 13,1 | 0,81 | 95,2 | 3 |
| 13 | – | 10 | 10 | 4,0 | 0,72 | 83,9 | 4 |
| 14 | – | 10 | 10 | 18,7 | 0,60 | 97,5 | 5A |
| 15 | – | – | – | 16,3 | 0,64 | 96,7 | 5B |
| 16 | 350 | 10 | 10 | 5,0 | 0,67 | 80,4 | 6 |
| 17 | 450 | – | 10 | 16,0 | 0,98 | 93,9 | 6 |
| 18 | 450 | – | 20 | 7,6 | 1,38 | 100 | 6 |
| 19 | 500 | – | 20 | 10,2 | 1,54 | 94,4 | 6 |
| 20 | 450 | – | 30 | 7,6 | 1,36 | 99,8 | 6 |
| 21 | 450 | 10 | 10 | 7,3 | 0,70 | 84,5 | 7A |
| 22 | – | – | – | 5,2 | 0,65 | 87,6 | 7B |
| V1 | 450 | 10 | 10 | 53,6 | 0,34 | 96,8 | 8 |
| V2 | – | – | – | 65,1 | 0,34 | 76,0 | 9 |
| V3 | – | – | – | 6,4 | 0,09 | 47,6 | 10 |
| V4 | – | – | – | 20,3 | 0,23 | 12,7 | 11 |
| Thermodyn. Gleichgewicht | 450 | – | – | – | 0,10 | – | – |

5

Tabelle 2

| Katalysator Nr. | Kristallgröße (µm) | p-DEB im Test % nach H.G. Karge | Binder | Modifizierung | Modul |
|---|---|---|---|---|---|
| Charakterisierung der in Tabelle 1 verwendeten Katalysatoren |||||| 
| 1 | 4,0 | 61 | Pseudoboehmit | | 36 |
| 2 | 8,5 | 88 | ohne | | 42 |
| 2 | 8,5 | 87 | ohne | | 42 |
| 2A | | 60 | | (-*) | 36 |
| 2B | | 92 | | (-*) | |
| 2C | | 74 | $SiO_2$ | (-*) | |
| 2D | | 64 | $SiO_2$ | (-*) | |
| 3 | 3,0 | 71 | ohne | 2,4 % Pd | 45 |
| 4 | 11,0 | 68 | ohne | | 51 |
| 5A | 3,0 | 84 | ohne | | 150 |
| 5B | | 93 | $SiO_2$ | | |
| 6 | 9,0 | 95 | $SiO_2$ | | 154 |
| 7A | 3,5 | 83 | ohne | | 554 |
| 7B | | 97 | $SiO_2$ | | |
| 8 | 0,2 | 34 | ohne | | 38,2 |
| 9 | 0,3 | 35 | ohne | | 72 |
| 10 | - | 40 | $SiO_2$ rein | | |
| 11 | - | 35 | $Al_2O_3$ rein | | |
| | | 28 | Thermodynamisches Gleichgewicht bei 250° C | | |

(*) Modifizierung mit $(NH_4)_2SiF_6$
Modul: molares Verhältnis von $SiO_2:Al_2O_3$
p-DEB: para-Diethylbenzol

EP 0 416 279 A1

Herstellung der Katalysatoren

Katalysator 1

Ein Aluminosilikatzeolith vom Pentasil-Typ wird unter hydrothermalen Bedingungen bei autogenem Druck und 150° C aus 650 g hochdispersem $SiO_2$, 203 g $Al_2(SO_4)_3$ x 18 $H_2O$ in einer Mischung aus 2,0 kg Wasser und 9,6 kg einer wäßrigen 1,6-Diaminohexan-Lösung (50 Gew.-% Amin) in einem Rührautoklaven hergestellt. Nach Abfiltrieren und Auswaschen wird das kristalline Reaktionsprodukt bei 110° C/24 h getrocknet und bei 500° C/24 h calciniert. Dieser Aluminosilikatzeolith enthält 80,4 Gew.-% $SiO_2$ und 3,8 Gew.-% $Al_2O_3$. Die Kristallgröße beträgt 4 $\mu$m.

Mit diesem Material werden durch Verformen mit Pseudoboehmit (9 Teile Zeolith : 1 Teil Pseudoboehmit) 2-mm-Stränge hergestellt, die bei 110° C 16 h getrocknet und bei 500° C 16 h calciniert werden.

Katalysatoren 2, 2A, 2B, 2C und 2D

Ein Aluminosilikatzeolith vom Pentasil-Typ wird unter hydrothermalen Bedingungen bei autogenem Druck und 184° C aus 700 g hochdispersem $SiO_2$ und 996 g $Al_2(SO_4)_3$ x 18 $H_2O$ in 12,2 kg einer wäßrigen Triethylentetramin-Lösung (38 Gew.-% Amin) in einem Rührautoklaven hergestellt. Nach Abfiltrieren und Auswaschen wird das kristalline Reaktionsprodukt bei 110° C/24 h getrocknet und bei 500° C/24 h calciniert. Dieser Aluminosilikatzeolith enthält 92,9 Gew.-% $SiO_2$ und 3,8 Gew.-% $Al_2O_3$; Modul 42). Die Kristallgröße beträgt 8.5 $\mu$m.

Mit diesem Material werden durch Verformen mit einem Verformungshilfsmittel 2-mm-Stränge hergestellt, die bei 110° C 16 h getrocknet und bei 500° C 16 h calciniert werden (Katalysator 2).

Katalysatoren 2 A und 2 B: Behandlung von Katalysator 2 mit $(NH_4)_2SiF_6$ und Trocknen bei 110° C (Katalysator 2 A) bzw. zusätzlich 4 Stunden Calcinieren bei 500° C in Gegenwart von Luft (Katalysator 2 B).

In einem Plastikbehälter werden 30 g des Katalysators 2 in 125 ml Wasser aufgeschlämmt. Dann werden 167 ml einer wäßrigen Lösung, welche 29.8 g des Hexafluorosilicats enthalten hinzugefügt. Der Behälter wurde geschlossen, auf 80° C erwärmt und 5 Stunden bei dieser Temperatur gehalten. Danach wurde filtriert und der Filterrückstand gründlich mit heißem Wasser (80° C) gewaschen. Der Filterkuchen wurde bei 110° C getrocknet und danach ein Teil des Zeolithen als Katalysator 2 A abgetrennt. Der Rest wurde 4 Stunden an der Luft bei 500° C calciniert (Katalysator 2 B).

Verstrangung des unbehandelten Pulvers von Katalysator 2 mit $SiO_2$

Das pulverförmige Material aus Katalysator 2 wird mit pyrogener Kieselsäure (8 Teile Zeolith : 2 Teile pyrogene Kieselsäure) zu 2-mm-Strängen verformt, die bei 130° C getrocknet und bei 500° C 16 h calciniert werden. Diese Stränge werden wie bei den Katalysatoren 2 A und 2 B mit Hexafluorosilicat behandelt und so die Katalysatoren 2 C (Trocknung 110° C) und 2 D erhalten (zusätzlich calciniert).

Katalysator 3

Ein Aluminosilikatzeolith vom Pentasil-Typ wird unter hydrothermalen Bedingungen bei autogenem Druck und 150° C über 85 h und anschließend 160° C über 85 h aus 650 g hochdispersem $SiO_2$, 203 g $Al_2(SO_4)_3$ x 18 $H_2O$ in einer Mischung aus 1,7 kg Wasser und 9,6 kg einer wäßrigen 1,6-Diaminohexan-Lösung (50 Gew.-% Amin) in einem Rührautoklaven hergestellt. Nach Abfiltrieren und Auswaschen wird das kristalline Reaktionsprodukt bei 110° C/24 h getrocknet und bei 500° C/24 h calciniert. Dieser Aluminosilikatzeolith enthält 81,4 Gew.-% SiO2 und 3,0 Gew.-% $Al_2O_3$. Die Kristallgröße beträgt 3 $\mu$m.

Mit diesem Material werden durch Verformen mit einem Verformungshilfsmittel 2-mm-Stränge hergestellt, die bei 110° C 16 h getrocknet und bei 500° C 16 h calciniert werden.

Die Stränge werden mit einer wäßrigen Pd(NO3)2-Lösung getränkt, bei 130° C getrocknet und bei 540° C calciniert. Der Pd-Gehalt beträgt 2,4 Massen-%.

Katalysator 4

Es wurde ein handelsüblicher ZSM-5-Katalysator (2-mm-Stränge) in der H-Form mit folgenden Eigenschaften eingesetzt:

| Katalysator | SiO$_2$/Al$_2$O$_3$ | Kristallitgröße ($\mu$m) |
|---|---|---|
| 4 | 52 | 11 |

Katalysator 5 A

Ein Aluminosilicatzeolith vom ZSM-5-Typ wird unter hydrothermalen Bedingungen bei autogenem Druck und 160°C aus 1,5 kg pyrogener Kieselsäure, 84,0 g Aluminiumsulfat-18-hydrat, 0,24 kg Natriumhydroxid, 0,98 kg Tetra-n-propylammoniumbromid und 18,0 kg wasser in einem Rührautoklaven hergestellt. Nach Abfiltrieren und Auswaschen wird das kristalline Reaktionsprodukt bei 110°C/24 h getrocknet und bei 500°C 24 h calciniert. Dieser Zeolith ist durch folgende Zusammensetzung charakterisiert: 85,4 Gew.-% SiO$_2$, 1,00 Gew.-% Al$_2$O$_3$, 0,75 Gew.-% Na. Die Kristallgröße beträgt 3 $\mu$m.

Mit diesem Zeolith werden durch Verformen mit einem Verformungshilfsmittel 2-mm-Stränge hergestellt, die bei 110°C/16 h getrocknet und bei 500°C 16 h calciniert werden.

Die Stränge werden viermal mit 20 gew.-%iger NH$_4$Cl-Lösung (15 ml Lösung/g Formkörper) bei 80°C in einem Steigrohr ausgetauscht. Anschließend wird chloridfrei gewaschen. Die Stränge werden bei 110°C 10 h getrocknet und anschließend bei 500°C 5 h calciniert. Der Na-Gehalt beträgt nun 0.015 Gew.-%.

Katalysator 5 B

Katalysator 5 B wird präpariert, indem man den ZSM-5-Zeolith aus Katalysator 5 A mit pyrogener Kieselsäure im Massenverhältnis 8 : 2 zu 2-mm-Strängen verformt, die 110°C getrocknet und bei 500°C 16 h calciniert werden.

Die Stränge werden viermal mit 20 Gew.-%iger NH4Cl-Lösung (15 ml Lösung/g Formkörper) bei 80°C in einem Steigrohr ausgetauscht. Anschließend wird chloridfrei gewaschen. Die Stränge werden bei 110°C 10 h getrocknet und anschließend bei 500°C 5 h calciniert. Der Na-Gehalt beträgt nun 0,012 Gew.-%.

Katalysator 6

Ein Aluminosilicatzeolith vom ZSM-5-Typ wird unter hydrothermalen Bedingungen bei autogenem Druck und 160°C aus 150 g pyrogener Kieselsäure, 8,4 g Aluminiumsulfat-18-hydrat, 24 g Natriumhydroxid, 98 g Tetra-n-propylammoniumbromid und 1,8 kg Wasser in einem Autoklaven hergestellt. Nach Abfiltrieren und Auswaschen wird das kristalline Reaktionsprodukt bei 110°C/24 h getrocknet und bei 500°C 24 h calciniert. Dieser Zeolith ist durch folgende Zusammensetzung charakterisiert: 86,2 Gew.-% SiO$_2$, 0,95 Gew.-% Al$_2$O$_3$, 0,67 Gew.-% Na. Die Kristallgröße beträgt 9 $\mu$m. Das Zeolithpulver wird viermal mit 20 Gew.-%iger NH$_4$Cl-Lösung (15 ml Lösung/g Zeolith) bei 80°C unter Rühren aufgeschlämmt. Anschließend wird chloridfrei gewaschen. Das Material wird bei 110°C getrocknet und anschließend bei 500°C 5 h calciniert. Der Na-Gehalt beträgt nun 0.01 Gew.-%. Mit diesem Zeolith werden durch Verformen mit pyrogener Kieselsäure (9 Teile Zeolith : 1 Teil Kieselsäure) 2-mm-Stränge hergestellt, die bei 110°C getrocknet und bei 500°C 16 h calciniert werden.

Katalysator 7 A

Ein Aluminosilicatzeolith vom ZSM-5-Typ wird unter hydrothermalen Bedingungen bei autogenem Druck und 160°C aus 1,5 kg pyrogener Kieselsäure, 34,0 g Aluminiumsulfat-18-hydrat, 0,22 kg Natriumhydroxid, 1,0 kg Tetra-n-propylammoniumbromid und 18,0 kg Wasser in einem Rührautoklaven hergestellt.

Nach Abfiltrieren und Auswaschen wird das kristalline Reaktions produkt bei 110°C/24 h getrocknet und bei 500°C 24 h calciniert. Dieser Zeolith ist durch folgende Zusammensetzung charakterisiert: 81,6 Ma-% SiO₂, 0,25 Ma-% Al₂O₃, 0,65 Ma-% Na. Die Kristallgröße beträgt 4 μm.

Mit diesem Zeolith werden durch Verformen mit einem Verformungshilfsmittel 2-mm-Stränge hergestellt, die bei 110°C/16 h getrocknet und bei 500°C 16 h calciniert werden.

Die Stränge werden viermal mit 20 gew.-%iger NH4Cl-Lösung (15 ml Lösung/g Formkörper) bei 80°C in einem Steigrohr ausgetauscht. Anschließend wird chloridfrei gewaschen. Die Stränge werden bei 110°C 10 h getrocknet und anschließend bei 500°C 5 h calciniert. Der Na-Gehalt beträgt nun 0.17Gew.-%.

Katalysator 7 B

Katalysator 7 B wird präpariert, indem man den ZSM-5-Zeolith aus Katalysator 7 A mit pyrogener Kieselsäure im Massenverhältnis 8 : 2 zu 2-mm-Strängen verformt, die 110°C getrocknet und bei 500°C 16 h calciniert werden.

Die Stränge werden viermal mit 20 gew.-%iger NH4Cl-Lösung (15 ml Lösung/g Formkörper) bei 80°C in einem Steigrohr ausgetauscht. Anschließend wird chloridfrei gewaschen. Die Stränge werden bei 110°C 10 h getrocknet und anschließend bei 500°C 5 h calciniert. Der Na-Gehalt beträgt nun 0.15 Gew.-%.

Katalysator 8 (Vergleichskatalysator)

Ein Aluminosilikatzeolith vom ZSM-11-Typ wird unter hydrothermalen Bedingungen bei autogenem Druck und 160°C aus 10,9 kg Wasser, 6,3 kg Natrium-Wasserglas (8,0 Gew.-% Na₂O, 26,5 Gew.-% SiO₂), 180 g Aluminiumsulfat-18-hydrat, 600 g Schwefelsäure konz. und 600 g 1,8-Diaminooctan in einem Rührautoklaven hergestellt. Nach Abfiltrieren und Auswaschen wird das kristalline Reaktionsprodukt bei 110°C 24 h getrocknet und bei 500°C 24 h calciniert. Dieser Zeolith ist durch folgende Zusammensetzung charakterisiert: 81,8 Gew.-% SiO₂, 1,82 Gew.-% Al₂O₃. Die Kristallgröße beträgt 0,2 μm.

Mit diesem Material werden durch Verformen mit einem Verformungshilfsmittel 2-mm-Stränge hergestellt, die bei 110°C 16 h getrocknet und bei 500°C 16 h calciniert werden.

Die Stränge werden viermal mit 20 gew.-%iger NH₄Cl-Lösung (15 ml Lösung/g Formkörper) bei 80°C in einem Steigrohr ausgetauscht. Anschließend wird chloridfrei gewaschen. Die Stränge werden bei 110°C 10 h getrocknet und anschließend bei 500°C 5 h calciniert. Der Na-Gehalt beträgt nun 0.015 Na-%.

Katalysator 9 (Vergleichskatalysator)

Ein Aluminosilikatzeolith vom Pentasil-Typ wird unter hydrothermalen Bedingungen bei autogenem Druck und 150°C aus 650 g hochdispersem SiO₂, 203 g Al₂(SO₄)₃ x 18 H₂O in 11,3 kg einer wäßrigen 1,6-Diaminohexan-Lösung (42 Gew.-% Amin) in einem Rührautoklaven hergestellt. Nach Abfiltrieren und Auswaschen wird das kristalline Reaktionsprodukt bei 110°C/24 h getrocknet und bei 500°C/24 h calciniert. Dieser Aluminosilikatzeolith enthält 92,6 Gew.-% SiO₂ und 4,6 Gew.-% Al₂O₃; Modul 38). Die Kristallgröße beträgt 0.3 μm.

Mit diesem Material werden durch Verformen mit einem Verformungshilfsmittel 2-mm-Stränge hergestellt, die bei 110°C 16 h getrocknet und bei 500°C 16 h calciniert werden.

Katalysator 10 (Vergleichskatalysator) ist ein handelsübliches Al₂O₃ (D 10-10®).

Katalysator 11 (Vergleichskatalysator) ist ein handelübliches SiO₂ (D 11-10®).

**Ansprüche**

1. Verfahren zur Herstellung von Kresolisomerengemischen mit einem Molverhältnis von para- zu meta-Kresol von 0,6:1 bis 10:1 durch Isomerisierung von Kresolgemischen oder ortho- und/oder meta-Kresol an Zeolithkatalysatoren des Pentasil-Typs in der Gasphase bei Temperaturen von 300 bis 600°C und Drücken von 0,01 bis 50 bar, dadurch gekennzeichnet, daß die mittlere Teilchengröße des Zeolithkatalysators mindestens 3 μm beträgt und die im festgelegten Test bei der Disproportionierung von Ethylbenzol zu Diethylbenzolisomeren eine Selektivität für para-Diethylbenzol von mindestens 60 % erreichen.

2. Verfahren nach Anspruch 1, dadurch gekennzeichnet, daß man Isomerengemische mit einem Molverhältnis von para- zu meta-Kresol von 0,6:1 bis 3:1 erhält.

3. Verfahren nach Anspruch 1, dadurch gekennzeichnet, daß man Zeolithe mit einer mittleren Teilchengröße von 3 bis 500 μm verwendet.

4. Verfahren nach Anspruch 1, dadurch gekennzeichnet, daß man Zeolithe mit einer mittleren Teilchengröße von 3 bis 50 μm verwendet.

5. Verfahren nach Anspruch 1, dadurch gekennzeichnet, daß man Zeolithe mit einer mittleren Teilchengröße von 3 bis 25 μm verwendet.

6. Verfahren nach Anspruch 1, dadurch gekennzeichnet, daß man Zeolithe mit einer mittleren Teilchengröße von 3 bis 15 μm verwendet.

7. Verfahren nach Anspruch 1, dadurch gekennzeichnet, daß man als Zeolithkatalysatoren Aluminosilikatzeolithe oder Galliumsilikatzeolithe des Pentasiltyps verwendet.

8. Verfahren nach Anspruch 1, dadurch gekennzeichnet, daß man Zeolithe des Pentasiltyps, welche mit Palladium und/oder Platin dotiert sind, verwendet.

9. Verfahren nach Anspruch 1, dadurch gekennzeichnet, daß man o-Kresol zur Isomerisierung einsetzt.

10. Verfahren nach Anspruch 1, dadurch gekennzeichnet, daß die Isomerisierung in der Gasphase bei bei Temperaturen von 350 bis 520 °C erfolgt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,Y | GB-A-2 012 271 (UNION RHEINISCHE BRAUNKOHLEN KRAFTSTOFF) <br> * Seite 1, Zeilen 22-29 * <br> — — — | 1 | C 07 C 37/00 <br> C 07 C 39/07 |
| D,Y | US-A-3 702 886 (R.J. ARGAUER et al.) <br> * Spalte 4, Zeilen 40-47 * <br> — — — | 1 | |
| D,Y | CHEMICAL SOCIETY SPECIAL PUBLICATION Band 33, 1980, Seiten 133-139, The Chemical Society, London, GB; G.T. KOKOTAILO et al.: "Pentasil Family of High Silica Crystalline Materials" <br> * Seite 133, Zusammenfassung * <br> — — — | 1 | |
| A | US-A-4 691 063 (D.J. ENGEL et al.) <br> * Ansprüche 1,4,7,8 * <br> — — — | 1 | |
| A | EP-A-0 191 120 (UOP) <br> * Ansprüche 1,7,8 *; & US-A-4503269 (Kat. D) <br> — — — | 1 | |
| A | DE-C-1 014 550 (PITTSBURGH CONSOLIDATION COAL CO.) <br> * Spalte 9, Zeilen 26-45 * <br> — — — | 1 | **RECHERCHIERTE SACHGEBIETE (Int. Cl.5)** |
| A | DE-A-2 005 153 (PITTSBURGH CONSOLIDATION COAL CO.) <br> * Ansprüche 1,7 * <br> — — — — | .1 | C 07 C 37/00 <br> C 07 C 39/00 |

Der vorliegende Recherchenbericht wurde für alle Patentansprüche erstellt

| Recherchenort | Abschlußdatum der Recherche | Prüfer |
|---|---|---|
| Berlin | 14 November 90 | PROBERT C.L. |